# EUROPÄISCHE PATENTSCHRIFT

(11) **EP 2 617 382 B1**
(45) Veröffentlichungstag und Bekanntmachung des Hinweises auf die Patenterteilung: **10.08.2016**
(21) Anmeldenummer: 13150569.5
(22) Anmeldetag: 08.01.2013
(51) Int. Cl.: A61C 17/20, A61C 3/06, A61C 3/00, A61C 3/03, A61C 5/02, A61C 17/06, A61B 17/3207

(54) **Dentalkürette**
Dental curette
Curette dentaire

(30) Priorität: 20.01.2012 DE 202012100225 U; 05.03.2012 DE 202012100778 U
(43) Veröffentlichungstag der Anmeldung: 24.07.2013
(73) Patentinhaber: Medizinische Hochschule Hannover, 30625 Hannover (DE)
(72) Erfinder: Günay, Hüsamettin, 30625 Hannover (DE)
(74) Vertreter: Taruttis, Stefan Georg

(56) Entgegenhaltungen:
- WO-A1-90/02524
- WO-A1-94/22380

## Beschreibung

HG-Kürette: Die vorliegende Erfindung betrifft eine zahnmedizinische Kürette für zahnmedizinische Zwecke, die auch als Dentalkürette bezeichnet wird, zur Entfernung von Ablagerungen auf Zähnen, einschließlich deren Wurzeloberfläche. Optional weist die Kürette einen Saugkanal auf, an dem eine Unterdruckquelle anordnbar ist. Mit Saugkanal wird die Kürette auch als HG-Saugkürette bezeichnet. Weiter optional kann die Kürette als Schallspitze ausgebildet sein, wobei ein Arbeitsende der Kürette mit Schall oder Ultraschall beaufschlagbar ist. In dieser Ausführungsform wird die Dentalkürette auch als HG-Schallspitze bezeichnet. Die erfindungsgemäße Kürette zeichnet sich durch eine Gestaltung aus, die für die Behandlung einer Mehrzahl unterschiedlicher Zähne geeignet ist, insbesondere in der Ausführungsform mit zwei symmetrisch angeordneten Arbeitsenden zur Behandlung aller Zähne eines menschlichen Gebisses im Ober- und Unterkiefer. Die Kürette ist für ein minimal-invasives, besonders effektives Entfernen von Belägen, einschließlich harter Beläge, sowie für die Glättung der Wurzeloberfläche bei vermindertem Verletzungsrisiko sowohl des behandelten Patienten als auch für den Behandler eingerichtet.

### Stand der Technik

Gattungsgemäße Küretten weisen an gegenüberliegenden Enden eines stabförmigen Griffs jeweils ein Behandlungsende auf, das in einer distalen sichelförmigen Spitze endet, die auf der dem Griff zugewandten Fläche eine Schneidkante aufweist und auf der dem Griff abgewandten Fläche abgerundet ist. Das sichelförmige Ende steht in einem Winkel von 80° bis 90° zur Längsachse des Griffs und ist mittels eines schmalen Trägers mit dem Griff verbunden, wobei der Träger die Position und Ausrichtung des sichelförmigen Endes zum Griff bestimmt. Der Träger weist angrenzend an das sichelförmige Ende üblicherweise einen Schaftabschnitt auf, der in einem spitzen Winkel oder etwa parallel zur Längsachse des Griffs ausgerichtet ist und zur Längsachse des Griffs versetzt ist, sodass das sichelförmige Ende etwa zentral zur Längsachse des Griffs angeordnet ist. Für die Anpassung an die unterschiedlichen Geometrien und Dimensionen einzelner Zähne wird ein Satz unterschiedlicher Küretten verwendet, die sich durch Größe und Neigung des sichelförmigen Endes und Länge und Neigung des Schafts unterscheiden.

Die WO-A-90/02524 beschreibt eine für die Chirurgie an der Wirbelsäule angepasste Ringkürette mit einem Griff, der durch einen gerade angesetzten Schaft mit einer endständigen Öse verbunden ist, die eine Schneidkante aufweist.

Die US 2005/0181328 A1 beschreibt Ultraschallaufsätze für zahnmedizinische Küretten, deren Arbeitsenden jeweils eine spitze Form aufweisen.

Die US 2005/0130102 A1 beschreibt eine zahnmedizinische Kürette, deren Arbeitsende die Form einer Spitze aufweist.

Die US 2008/0318184 A1 beschreibt Aufsätze für zahnmedizinische Küretten, deren Arbeitsenden jeweils sichelförmig gebogen sind und eine scharfe Spitze aufweisen.

Die US 5,145,368 beschreibt eine Saugkürette, deren Arbeitsende ein schräg geschnittenes Rohr ist, oder ein bogenförmiges ausgeklinktes Rohr, das eine Kralle mit zentraler Saugöffnung bildet.

An bekannten Küretten ist nachteilig, dass das sichelförmige Ende, das einseitig oder beidseitig eine Schneide aufweist, in einer Spitze ausläuft, die ein Verletzungsrisiko sowohl für den behandelten Patienten, insbesondere dessen Zahnfleisch bildet, als auch für den Behandler, z.B. bei gleichzeitigem Arbeiten mit einer freien Hand in der Nähe des Arbeitsendes der Kürette. Weiter nachteilig ist, dass für die Reinigung eines vollständigen menschlichen Gebisses, bzw. von dessen Wurzeloberflächen, ein Satz von 7 bis 9 doppelendigen Instrumenten erforderlich ist.

### Aufgabe der Erfindung

Der Erfindung stellt sich die Aufgabe, eine alternative Kürette bereitzustellen, insbesondere eine Kürette, die ein effektives Entfernen harter und weicher Beläge von Zähnen erlaubt und ein Arbeitsende aufweist, das ein drastisch reduziertes Verletzungsrisiko hat. Weiter bevorzugt soll die Kürette durch ihre Gestaltung an die Reinigung einer Vielzahl von unterschiedlichen Zahngeometrien angepasst sein, sodass für die vollständige Behandlung bzw. Reinigung eines vollständigen Gebisses vorzugsweise maximal drei, bevorzugter zwei Küretten unterschiedlicher Dimensionen oder Geometrien, besonders bevorzugt 1 Kürette einsetzbar ist.

### Beschreibung der Erfindung

Die Erfindung löst die Aufgabe mit den Merkmalen der Ansprüche, insbesondere mit einer Dentalkürette, die zumindest an einem Ende, vorzugsweise an beiden Enden eines Griffs jeweils ein Arbeitsende aufweist, das distal eine Öse mit zumindest einer Schneidkante aufweist. Das Arbeitsende, das distal zum Griff die Öse aufweist, weist zwischen dem Griff und der Öse angrenzend an den Griff einen Distanzabschnitt, angrenzend an die Öse einen Schaftabschnitt und zwischen dem Distanzabschnitt und dem Schaftabschnitt einen Winkelabschnitt auf.

Alternativ kann an dem dem Winkelabschnitt gegenüberliegenden Ende des Distanzabschnittes ein Befestigungsstück angeordnet sein, das lösbar an dem Griff anordnbar ist.

Generell sind der Schaftabschnitt, der Winkelabschnitt und der Distanzabschnitt vorzugsweise einstückig ausgebildet, weiter bevorzugt weisen diese Abschnitte eine glatte Oberfläche und ausschließlich kontinuierliche Übergänge auf. Der Winkelabschnitt, der an den Schaftabschnitt angrenzt, ist in einem stumpfen Winkel mit dem Schaftabschnitt verbunden, beispielsweise in einem Winkel von 110 bis 165°, vorzugsweise 120 - 150°, bevorzugter 135°, wobei gegenüber dem Schaftabschnitt der Winkelabschnitt an den Distanzabschnitt in einem Winkel angrenzt, der ebenfalls stumpf oder gestreckt ist, beispielsweise in einem Winkel von 140 - 180°. Bevorzugt ist die Öse etwa in der Längsmittelachse des Griffs angeordnet.

Der Distanzabschnitt und der Winkelabschnitt können gemeinsam einen Bogen bilden, der den Schaftabschnitt mit dem Griff verbindet. Alternativ können der Distanzabschnitt und der Winkelabschnitt auch in einem gestreckten Winkel zueinander angeordnet sein, sodass sie ohne wahrnehmbaren Übergang ineinander übergehen.

In der Ausführungsform, in der der Träger zusätzlich zu dem Schaftabschnitt, dem Winkelabschnitt und dem Distanzabschnitt ein Befestigungsstück aufweist, das gegenüber dem Winkelabschnitt an dem Distanzabschnitt angeordnet ist, verbindet das Befestigungsstück den Distanzabschnitt mit dem Griff, insbesondere mit dem im Griff enthaltenen Vibrationserzeuger. Dabei ist es bevorzugt, dass der Griff einen Vibrationserzeuger aufweist, insbesondere einen luftbetriebenen Vibrationserzeuger, der eingerichtet ist, Vibrationen im Schall- und Ultraschallbereich, insbesondere im Bereich von 6000 bis 32000 Hz, z.B. im Bereich von 6000 bis 11000 Hz oder im Bereich von 6000 bis 8000 Hz, auch als Schall bezeichnet, oder im Bereich von 25000 bis 30000 Hz, auch als Ultraschall bezeichnet, zu erzeugen. Der Griff mit dem Vibrationserzeuger wird auch als Schallhandstück bezeichnet. Das Befestigungsstück, das Teil des Trägers ist, ist bevorzugt schwingungsfest mit dem Schallhandstück, insbesondere mit dem Vibrationserzeuger, verbindbar. Der Vibrationserzeuger ist dazu eingerichtet, das Arbeitsende der Kürette, insbesondere den Träger mit der am distalen Ende angeordneten Öse, mit Vibrationen (Schall) zu beaufschlagen, was eine leichtere und effektivere Reinigung der Zähne erlaubt. Insbesondere wird das Arbeitsende der Kürette mit Vibrationen bei Frequenzen zwischen 6000 bis 8000 Hz oder mit Vibrationen bei Frequenzen zwischen 6000 und 32000 Hz beaufschlagt.

Die Gestaltung der Kürette erlaubt die Hartgewebe und Weichgewebe schonende blutungsarme Wurzelreinigung an allen Zähnen. Bevorzugt erlaubt die Kürette eine effektive Reinigung und Glättung von Wurzeloberflächen aller Zähne. Die Küretten haben den Vorteil, dass 3, vorzugsweise 2 Größen ausreichen, um ein vollständiges menschliches Gebiss, bzw. dessen Wurzeloberflächen, zu reinigen. Dabei unterscheiden sich die Küretten z.B. nur hinsichtlich der Größen des Trägers. Entsprechend betrifft die Erfindung in einer Ausführungsform einen Satz von 3, bevorzugt von 2 Küretten, die an einem Griff zwei gegenüberliegende Arbeitsenden unterschiedlicher Dimensionen aufweisen, die zur vollständigen Reinigung und Glättung der Wurzeloberflächen eines vollständigen menschlichen Gebisses ausreichen.

Das Arbeitsende der erfindungsgemäßen Kürette aus einem am Griff etwa koaxial zur Längsachse des Griffs angeordneten Distanzabschnitt, einem gegenüber dem Griff und Distanzabschnitt in einem Winkel zum Distanzabschnitt angeordneten Winkelabschnitt und einem Schaftabschnitt, der in einem stumpfen Winkel zum Winkelabschnitt steht, erlaubt die Positionierung der Öse etwa um die Längsmittelachse des Griffs, wobei Distanzabschnitt, Winkelabschnitt und Schaftabschnitt einen Träger für die Öse bilden, der zur Behandlung von Seitenflächen eines Zahns einschließlich des Wurzelbereichs im Mund eines Patienten und eine gegen Verbiegung stabile Verbindung der Öse mit Griff erlaubt, die die Zahnkrone umgeht, bzw. eine Ausnehmung für die Zahnkrone entlang der Längsmittelachse des Griffs bildet.

Der Distanzabschnitt, der Winkelabschnitt und der Schaftabschnitt sowie das optionale Befestigungsstück, aus denen der Träger für die Öse gebildet ist, sind in einer gemeinsamen Längsmittelebene angeordnet, durch die die Längsmittelachse des Griffs verläuft. Die Ebene, in der sich die Öse erstreckt und ihre Öffnung aufspannt, ist gegen die gemeinsame Längsmittelebene des Trägers geneigt, in einem Winkel von 30 - 60°, bevorzugt 40 - 50°, bevorzugter 45°, wobei bei einer Kürette mit einem Arbeitsende an jedem Griffende die jeweils endständigen Ösen entlang der Mittelebene in Winkeln geneigt sind, die in Bezug zur Längsmittelebene zueinander parallel liegen. Dass die Öse eine Öffnung aufspannt, bedeutet, dass jeweils gegenüberliegende Kanten der Öse zueinander beanstandet sind und in einer Ebene angeordnet sind, die geneigt ist, z.B. gegen die gemeinsame Längsmittelebene von Distanzabschnitt, Winkelabschnitt und Schaftabschnitt, bzw. gegen die gemeinsame Längsmittelebene des Trägers. Die Gesamtheit aller gegenüberliegender Kanten der Öse begrenzt dabei den Querschnitt der Öse.

Bevorzugt sind die Ösen bei einer Kürette, die an beiden Enden des Griffs je ein Arbeitsende aufweist, parallel zueinander zur gemeinsamen Längsmittelebene geneigt, um in der Aufsicht auf den Griff nach Drehung des Griffs um eine Achse, die senkrecht zu seiner Längsachse, insbesondere in der Längsmittelebene liegt, in gegenüberliegenden Neigungen angeordnet zu sein, z.B. eine Öse mit Neigung nach links zur Längsmittelebene des Griffs und die Öse am anderen Ende des Griffs nach der Drehung der Kürette um diese Achse in der Längsmittelebene mit Neigung nach rechts zu bilden. Besonders bevorzugt sind die Träger zweier an gegenüberliegenden Enden des Griffs angeordneter Arbeitsenden spiegelsymmetrisch zum Mittelpunkt des Griffs angeordnet, wobei weiter bevorzugt die Arbeitsenden gleich dimensioniert sind.

In einer Ausführungsform, ist der Träger aus Distanzabschnitt, Winkelabschnitt und Schaftabschnitt einstückig mit der Öse ausgebildet, besonders bevorzugt auch einstückig mit dem Griff.

In einer weiteren Ausführungsform, in der der Griff einen Vibrationserzeuger zur Beaufschlagung der Öse mit Vibrationen (Schall oder Ultraschall) aufweist, der bevorzugt an einem Befestigungsstück anordnbar ist, ist der Träger aus Distanzabschnitt, Winkelabschnitt, Schaftabschnitt und Befestigungsstück einstückig mit der Öse ausgebildet. Das Befestigungsstück ist kraftschlüssig und/oder formschlüssig mit dem Vibrationserzeuger in Eingriff bringbar, insbesondere ist es mit dem Vibrationserzeuger verrastbar oder verklemmbar oder mittels einer Bajonettkupplung mit diesem verbindbar.

Der Vibrationserzeuger ist dazu eingerichtet, Schall bzw. Ultraschall zu erzeugen und auf das Arbeitsende der Kürette zu übertragen. Dazu ist der Vibrationserzeuger schwingungsfest mit dem Träger, insbesondere mit dem Verbindungsstück verbunden, wobei das Verbindungsstück an dem dem distalen Ende des Trägers gegenüberliegenden Ende angeordnet ist. Dabei ist es bevorzugt, dass der Vibrationserzeuger und das Verbindungsstück über eine form- und/oder kraftschlüssige Verbindung miteinander verbunden sind, beispielsweise mittels eines Gewindes. Besonders bevorzugt bildet der Vibrationserzeuger einen Teil des Griffs und ist innerhalb des Griffs angeordnet, beispielsweise innerhalb eines an den Träger angrenzenden Griffstücks.

In der Ausführungsform, in der der Griff Teil eines Schallhandstücks bzw. Ultraschallhandstücks ist und dementsprechend einen Vibrationserzeuger aufweist, kann entlang des Griffs und entlang des Trägers ein Wasserführungskanal angeordnet sein, dessen Mündung in dem Bereich der Öse oder des Schaftabschnitts angeordnet ist, wobei der Wasserführungskanal gegenüber seiner Mündung einen Anschluß für Wasser aufweist. Bei einer Mündung in dem Bereich des Schaftabschnitts können die Mündungsöffnungen an jeder beliebigen Position innerhalb des Schaftabschnitts angeordnet sein, z.B. auf der Seite, an der der Distanzabschnitt und der Winkelabschnitt in einem stumpfen Winkel mit dem Schaftabschnitt verbunden sind, oder an der Seite, an der der Distanzabschnitt und der Winkelabschnitt in einem überstumpfen Winkel mit dem Schaftabschnitt verbunden sind. Alternativ können auch eine oder mehrere Mündungsöffnungen des Wasserführungskanals auf jeder dieser Seiten angeordnet sein. Die Anordnung eines Wasserführungskanals entlang des Trägers bzw. entlang des Griffs der Kürette erlaubt eine Spülung vor, während oder nach der Zahnreinigung und erlaubt weiterhin eine Kühlung. Für eine kontrollierte Wasserzufuhr ist der Wasserführungskanal an seinem Anschluss für Wasser mit einer Wasserversorgung verbunden, vorzugsweise mit einer Wasserpumpe.

Generell bevorzugt weist die Dentalkürette Markierungen gemäß der WHO-Vorschrift zur Sondeneinteilung auf. Bevorzugt weist der Schaftabschnitt zumindest eine Markierung auf, bevorzugt eine erste Markierung, die insbesondere der WHO-Sondeneinteilung entspricht, die in einem Abstand von 3,5 bis 5,5 mm, in einem Abstand von 3 bis 12 mm oder 3,5 bis 12 mm von der distalen Kante der Öse angeordnet ist. Bevorzugt weist der Schaftabschnitt eine erste Markierung in einem Abstand von ca. 10 bis 12 mm und eine optionale zweite Markierung auf, die in einem Abstand von ca. 8,5 - 9 mm von der distalen Kante der Öse angeordnet ist und/oder eine dritte Markierung, die in einem Abstand von ca. 3,5 bis 5,5 mm, bevorzugt etwa 3,5 mm von der distalen Kante der Öse angeordnet ist.

Die erste und die zweite Markierung können sich z.B. über einen Bereich von 0,5-2 mm, bevorzugt 1-1,5 mm erstrecken und die dritte Markierung erstreckt sich vorzugsweise über einen Bereich von 0,5-3 mm, bevorzugter 1,5-2,5 mm, bevorzugter 2 mm, jeweils längs des Schaftabschnitts.

Der Distanzabschnitt weist vorzugsweise eine Länge von 12 -16 mm, bevorzugt 14 mm auf, der Winkelabschnitt weist vorzugsweise eine Länge von 9 -12 mm, bevorzugt 10 mm auf, und der Schaftabschnitt erstreckt sich einschließlich der Öse bis zu deren distaler Kante über eine Länge von z.B. 8 -14 mm, bevorzugt 10 - 12 mm, bevorzugter 12 mm.

Die Öse hat vorzugsweise einen Außendurchmesser von 2 - 5 mm, bevorzugt von 3 mm.

Die Öse weist zumindest an einer, vorzugsweise an beiden von zwei gegenüberliegenden Kanten eine zumindest anteilige Schneidkante auf, die die beabstandeten Öffnungen der Öse begrenzt. Vorzugsweise sind die gegenüberliegenden bzw. beabstandeten Schneidkanten einer Öse in parallelen Ebenen angeordnet, bevorzugter sind die beabstandeten Schneidkanten zueinander parallel bzw. aus Sicht auf die Öffnung der Öse deckungsgleich. Die Öse ist umfänglich geschlossen; bevorzugt ist jede Schneidkante umlaufend um die Öse angeordnet bzw. umlaufend geschlossen.

Die zumindest eine Schneidkante der Öse wird von einem Querschnitt der Öse gebildet, dessen Außenflächen eben oder konvex sind, vorzugsweise halbkreisförmig oder parabelförmig, tropfenförmig, wahlweise kegelstumpfförmig, und dessen zum Inneren der Öse gewandte Oberfläche, auch als Innenfläche bezeichnet, eben, konvex oder konkav, z.B. mit aneinander grenzenden kreisförmigen ebenen Flächen oder gebogenen Flächen gebildet ist. Die Innenfläche der Öse läuft in einem Winkel von ca. 120 - 70°, bevorzugt 110 - 80°, bevorzugter 80 - 90° auf die Außenfläche der Öse, sodass jede Schneidkante von der Innenfläche der Öse und ihrer Außenfläche gebildet wird. Die Schneidkante erstreckt sich vorzugsweise zumindest entlang der Hälfte der Öse, die dem Schaftabschnitt gegenüber liegt, bevorzugter erstreckt sich die Schneidkante entlang der gesamten Öse. Durch die Anordnung der Schneidkante entlang der Ebene, in der die Öse ihre Öffnung aufspannt, vorzugsweise entlang beider durch den Querschnitt der Öse beabstandeter Ebenen, in denen die Öse ihre Öffnung aufspannt, ist die Öse geeignet, Zahnbeläge durch Bewegen der Öse etwa parallel zu der zu reinigenden Zahnoberfläche geeignet. Durch die Anordnung der Schneidkante entlang einer oder beider Kanten, die die Ösenöffnung begrenzen, weist das Arbeitsende der erfindungsgemäßen Kürette keine Spitze auf, die zu Verletzungen führen kann. Es hat sich hingegen gezeigt, dass die Anordnung der Schneidkante zumindest anteilig entlang der Öse eine effektive Entfernung von Belägen von Zahnoberflächen erlaubt. Diese an die Öffnung der Öse angrenzende eine oder zwei um die Innenfläche der Öse beabstandeten Schneidkanten machen die Kürette insbesondere als Ziehinstrument geeignet, das mittels Ziehen über eine zu behandelnde Oberfläche bewegt wird. In Ausführungsformen mit Vibrationsgenerator wird ein noch effizienteres Entfernen von Zahnbelägen mit der erfindungsgemäßen Dentalkürette erlaubt. Die bei der Reinigung entfernten Zahnbeläge werden bevorzugt während des Reinigungsprozesses durch die Zuführung von Wasser durch den Wasserführungskanal mit daran angeschlossener Wasserversorgung weggespült.

Generell liegt ein Vorteil der erfindungsgemäßen Kürette darin, dass die Schneidkanten durch aneinanderstoßende, vorzugsweise in sich geschlossene Flächen gebildet werden, die in einem Winkel von 70 bis 120°, vorzugsweise 80 bis 110°, besonders bevorzugt etwa 90° ±5° zueinander angeordnet sind. Dies vermeidet beispielsweise ein Einschneiden in Weichgewebe, wie dies bei herkömmlichen Schneidkanten von Messern erfolgt, die Schneidkanten mit spitzen Winkeln aufweisen.

In einer ersten Ausführungsform ist der Winkelabschnitt im Wesentlichen kolinear mit dem Distanzabschnitt angeordnet, sodass der Schaftabschnitt in einem stumpfen Winkel zum Winkelabschnitt und Distanzabschnitt angeordnet ist, die z.B. im Wesentlichen entlang einer Linie verlaufen. In dieser Ausführungsform ist bevorzugt der Träger aus Distanzabschnitt, Winkelabschnitt und Schaftabschnitt mit einem innenliegenden Wasserführungskanal ausgebildet, dessen eines Ende in dem Bereich der Öse oder des Schaftabschnitts mündet. Der Wasserführungskanal erstreckt sich zumindest abschnittsweise innerhalb des Schallhandstücks in Richtung auf das der Öse gegenüberliegende Ende. Vorzugsweise weist der Distanzabschnitt oder der Griff eine mit diesem Wasserführungskanal verbundene Öffnung auf. Der Wasserführungskanal ist in einem Abstand gegenüber seiner Mündung, die innerhalb des von der Öse oder von dem Schaftabschnitt umfassten Bereichs angeordnet ist, mit einer Wasserquelle verbunden, vorzugsweise an dem Ende des Schallhandstücks, das dem Arbeitsende der Kürette gegenüber liegt.

Die Ausführungsform ohne Vibrationserzeuger, die mit Saugkanal auch als HG-Saugkürette bezeichnet wird, eignet sich zum Absaugen von Flüssigkeit aus Zahnfleischtaschen, insbesondere angrenzend an den Zahnwurzelbereich. Die mit dem Kanal verbundene Öffnung dient der Saugkraftregulierung mittels des Fingers eines Behandlers, da ein Abdecken der Öffnung den Unterdruck bis zur Mündung des Kanals innerhalb der Öse wirken lässt, während eine nicht abgedeckte Öffnung die Wirkung des Unterdrucks an der Mündung des Kanals innerhalb der Öse signifikant verringert. Der Saugkanal weist an einem Ende gegenüber seiner Mündung einen Anschluß für einen Saugschlauch auf.

Vorzugsweise weist die Kürette dieser Ausführungsform einen biegsamen Stößel auf, der nach Verwendung der Kürette zum Absaugen für die Reinigung in den Saugkanal geschoben wird, z.B. um Beläge durch die Mündung des Saugkanals zu schieben.

In dieser Ausführungsform ist bevorzugt, dass zwei HG-Saugküretten in einem Satz vorliegen, eine mit einer Neigung der Öse gegen die Längsmittelebene aus Distanzabschnitt und Schaftabschnitt in einem Winkel von 30 - 60°, vorzugsweise ca. 45°, und die andere mit einer spiegelbildlich entgegengesetzten Neigung der Ebene, in der die Öse angeordnet ist, zur Längsmittelebene aus Distanzabschnitt, Winkelabschnitt und Schaftabschnitt des Trägers. Ein solcher Satz weist eine Saugkürette auf, die in der Aufsicht auf den Griff ein Arbeitsende mit Neigung in eine Richtung, z.B. nach rechts hat und eine Saugkürette, deren Arbeitsende mit entgegengesetzter Neigung, z.B. nach links angeordnet ist, jeweils in Bezug zur Längsmittelebene. Ein solcher Satz zweier HG-Saugküretten, deren Arbeitsenden spiegelbildlich zur Längsmittelebene geneigt sind, ist an die Verwendung für gegenüberliegende Zahnfleischtaschen angepasst.

In einer weiteren Ausführungsform weist die Kürette bevorzugt einen massiven Träger auf, und entsprechend keinen Saugkanal.

In einer Variante weist die Öse zumindest über einen Abschnitt ihres Außenumfangs eine Schneidkante auf, die dadurch gebildet ist, dass der Querschnitt der Öse entlang des Außenumfangs kegelstumpfförmig ist, wobei die Stirnfläche des kegelstumpfförmigen Querschnitts, die den äußeren Umfang der Öse bildet, in einem Winkel, z.B. von 70 - 120°, bevorzugt 85 - 95° gegen die Außenflächen des kegelstumpfförmigen Querschnitts angeordnet ist.

In dieser Ausführungsform eignet sich die Kürette zum Abtragen von Belägen besonders durch Schieben bzw. Ausüben von Druck aus Richtung des Griffs gegen eine Zahnoberfläche, insbesondere etwa parallel zu einer Zahnoberfläche, so dass die Kürette dieser Ausführungsform auch zur Verwendung als Stoßinstrument geeignet ist.

Die Erfindung wird nun genauer in Bezug auf die Figuren beschrieben, die
- in Figur 1 das Arbeitsende einer Kürette,
- in Figur 2 eine erste Ausführungsform der Kürette,
- in Figur 3 eine zweite Ausführungsform der Kürette,
- in Figur 4 das Arbeitsende einer Kürette,
- in Figuren 5a und 5b Formen der Öse des Arbeitsendes der Kürette,
- in Figuren 6 bis 15 beispielhafte Querschnitte der Öse entlang A-A von Figur 5,
- in Figur 16 das Arbeitsende einer Kürette (Schallspitze mit einer Neigung nach rechts)
- in Figur 17 ein weiteres Arbeitsende einer Kürette (Schallspitze mit einer Neigung nach links) und
- in Figur 18 ein weiteres Arbeitsende einer Kürette zeigen.

In den Figuren bezeichnen gleiche Bezugsziffern funktionsgleiche Elemente.

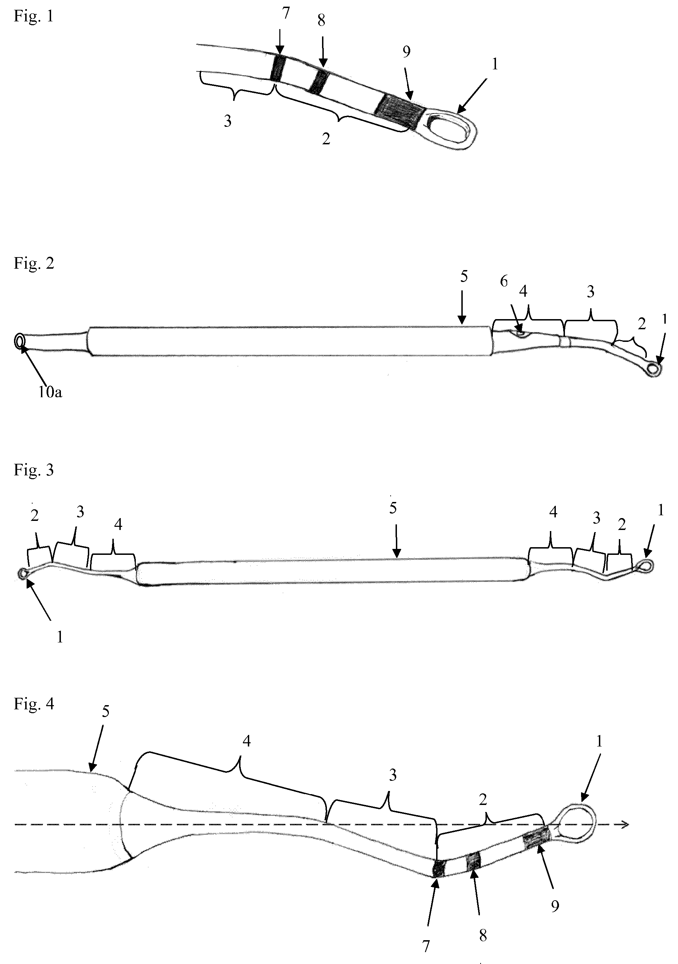

Figur 1 zeigt vom Arbeitsende einer Kürette die distal von einem Griff 5 angeordnete Öse 1, die mit einem Schaftabschnitt 2 verbunden ist. Gegenüber der Öse 1 ist der Schaftabschnitt 2 in einem stumpfen Winkel von ca. 120° mit einem Winkelabschnitt 3 verbunden, der in einen Distanzabschnitt 4 übergeht, wobei vorliegend der Winkelabschnitt 3 im Wesentlichen linear oder in einem leichten Bogen in den Distanzabschnitt 4 übergeht. Der Distanzabschnitt 4 ist gegenüber der Öse 1 mit einem Griff verbunden.

Figur 2 zeigt die erste Ausführungsform (HG-Saugkürette), in der die Öse 1 mittels des Schaftabschnitts 2 und des in einem flachen Winkel angeordneten Winkelabschnitts 3 und dem Distanzabschnitt 4 an einem Griff 5 angeordnet ist, wobei die Ebene, in der die Öse 1 aufgespannt ist, in einem Winkel von ca. 45° zu der Längsmittelebene geneigt ist, in der sich die Längsachse des Griffs 5, der darin angeordnete Distanzabschnitt 4, der Winkelabschnitt 3 und der Schaftabschnitt 2 erstrecken. Die Ösen 1 an gegenüberliegenden Enden des Griffs 5 sind vorzugsweise in parallelen Ebenen geneigt, so dass sie bei Drehung des Griffs 5 um eine Senkrechte zu seiner Längsachse in der Längsmittelebene in gegenüberliegenden Neigungen angeordnet sind, z.B. eine Öse 5 nach rechts, eine nach dieser Drehung nach links zur Längsmittelebene.

In dieser Ausführungsform, ist bevorzugt, dass ein Saugkanal 10 längs des Griffs 5 und des Trägers aus Distanzabschnitt 4, Winkelabschnitt 3 und Schaftabschnitt 2 verläuft, der innerhalb der Öse 1 mündet. Zur Regulierung der Saugleistung, die durch den Unterdruck, der sich entlang des Saugkanals erstreckt und in der Öse 1 wirkt, weist diese Ausführungsform vorzugsweise eine Öffnung 6 auf, die mit dem Saugkanal verbunden ist. Die Öffnung 6 kann beispielsweise im Distanzabschnitt 4 oder im Griff 5 angeordnet sein.

Figur 3 zeigt eine Aufsicht auf die zweite Ausführungsform der Kürette, die wie bevorzugt an beiden Enden eines stabförmigen Griffs 5 jeweils eine distal angeordnete Öse 1 aufweist, die mit einem Träger aus dem Schaftabschnitt 2, einem daran in einem flachen Winkel gegenüber der Öse 1 angeordneten Winkelabschnitt 3 und einem Distanzabschnitt 4 mit dem Griff 5 verbunden ist.

Figur 4 zeigt ausschnittsweise einen Griff 5, der mit dem Distanzabschnitt 4 verbunden ist, der in einem Bogen in den Winkelabschnitt 3 übergeht. Der Winkelabschnitt 3 ist in einem stumpfen Winkel mit dem Schaftabschnitt 2 verbunden, der gegenüber des Winkelabschnitts 3 an die Öse 1 angrenzt. Die für den Distanzabschnitt 4, den Winkelabschnitt 3 und den Schaftabschnitt 2 einschließlich der Öse 1 angegebenen Größen sind beispielhaft. Diese Darstellung macht deutlich, dass bevorzugt die Öse 1 im Bereich der Längsachse des Griffs 5 angeordnet ist, während der Winkelabschnitt 3 und der Schaftabschnitt 2 in einem Abstand zur Längsachse des Griffs 5 angeordnet sind und eine Ausnehmung gegenüber der Längsachse des Griffs 5 bilden. Diese Ausnehmung dient bei der Anordnung der Öse 1 an einer Zahnoberfläche zur Aufnahme bzw. Umgehung einer Zahnkrone, die längs der Längsachse des Griffs 5 positioniert ist.

Die Öse 1 erstreckt sich wie in einer Ebene, die gegenüber der Längsmittelebene in einem Winkel von 30 bis 60° geneigt ist, während der Träger aus Distanzabschnitt 4, Winkelabschnitt 3 und Schaftabschnitt 2 in der Längsmittelebene angeordnet ist, die sich entlang der Längsachse des Griffs 5 erstreckt.

Figur 4 zeigt auch die bevorzugten Markierungen des Schaftabschnitts 2 anhand einer ersten Markierung 7, die beispielsweise eine vorbestimmte maximale Eindringtiefe der Öse 1 in eine Zahnfleischtasche anzeigt, eine zweite Markierung 8, die beispielsweise eine zweite maximale Eindringtiefe in eine Zahnfleischtasche für einen anderen Zahn als die erste Markierung 7 anzeigt, sowie eine dritte Markierung 9, die beispielsweise eine maximale Eindringtiefe für die Öse 1 in eine Zahnfleischtasche eines weiteren Zahns anzeigt.

Die beispielhaft angegebenen Dimensionierungen für den Distanzabschnitt 4, den Winkelabschnitt 3 und den Schaftabschnitt 2 einschließlich der Öse 1, sowie die Positionierung der Markierungen 7, 8, 9 entlang des Schaftabschnitts 2 sind bevorzugt an die Dimensionen eines erwachsenen menschlichen Gebisses angepasst.

Weiterhin zeigt Figur 4 die bevorzugte Anordnung der Öse 1 auf der Längsachse des Griffs 5, wobei Schaftabschnitt 2, Winkelabschnitt 3 und Distanzabschnitt 4 in einer gemeinsamen, durch die Längsachse des Griffs 5 angeordnete Längsmittelebene angeordnet sind.

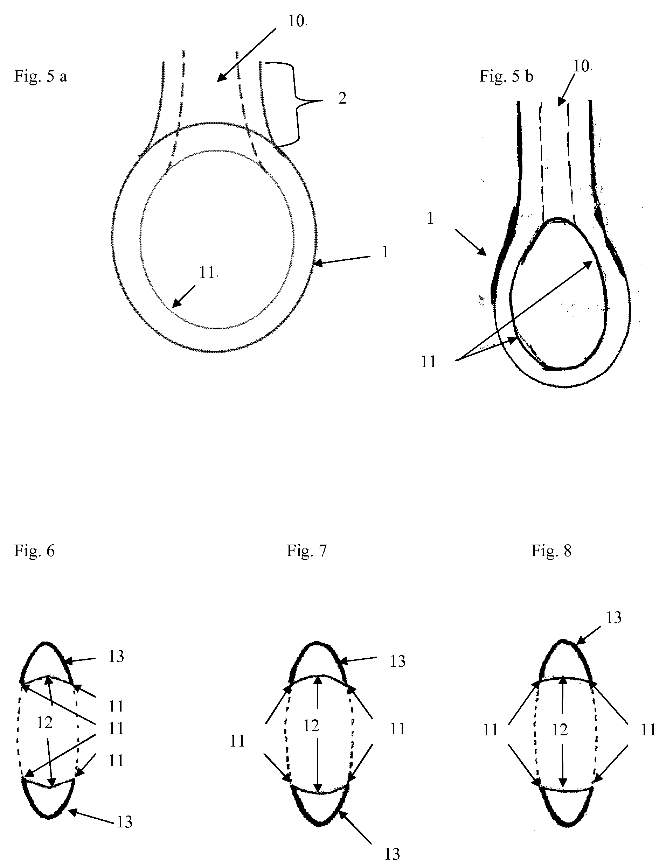

Die Figuren 5a und 5b zeigen jeweils eine schematische Aufsicht auf eine Öse 1 mit angrenzendem Schaftabschnitt 2. Die in Figur 5a gezeigte Öse 1 ist kreisförmig bis oval, die Öse 1 von Figur 5b tropfenförmig. Der optionale, für die erste Ausführungsform geeignete Saugkanal 10 ist innerhalb des Schaftabschnitts 2 angeordnet und mündet in der Innenfläche 12 der Öse 1.

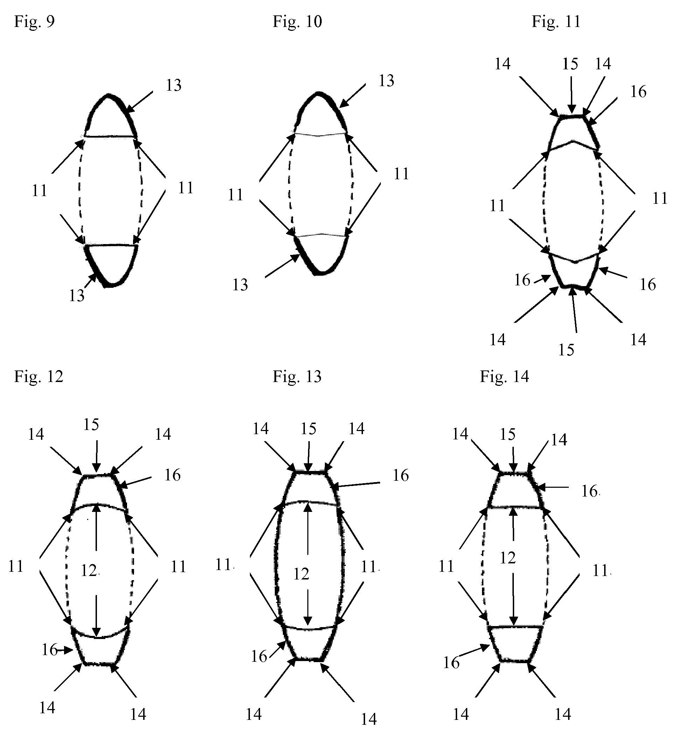

Die Figuren 6 bis 15 zeigen schematisch die Öse 1 im Schnitt entlang A-A von Figur 5 in der bevorzugten Ausführungsform, in der die Öse 1 jeweils eine umlaufende Schneidkante aufweist, die die beiden beabstandeten Öffnungen der Öse 1 begrenzen. Der Querschnitt der Öse 1 weist eine der Mitte der Öse 1 zugewandte Innenfläche 12 auf, die in einem Winkel gegen die bogenförmig gewölbte Außenfläche 13 stößt und jeweils umlaufende Schneidkanten 11 bildet. Diese umlaufenden Schneidkanten 11 erlauben die Verwendung der Kürette als Ziehinstrument. Die Innenfläche 12 kann konvex sein und zwei in sich ebene umlaufende, in einem Winkel aneinander stoßende Teilflächen aufweisen, wie in Figur 6 gezeigt ist, oder bogenförmig konkav ausgebildet sein, wie in Figuren 7 und 8 dargestellt ist. Figur 9 zeigt eine einfache Ausführungsform, bei der die Innenfläche 12 zylindrisch ist, beispielsweise als Bohrung hergestellt.

Figur 10 zeigt eine Ausführungsform, bei der die Innenfläche 12 konkav ausgebildet ist, dass sie beispielsweise etwa senkrecht auf die Außenfläche 13 stößt. Auch bei konvexer Innenfläche 12 kann die Innenfläche 12 bogenförmig oder durch zwei aneinander grenzende in sich ebene ringförmige Teilflächen ausgebildet sein.

Die in Figuren 6 bis 10 gezeigten Ausführungsformen der Öse 1 bilden nur Schneidkanten, die an die Öffnung der Öse 1 angrenzen. Diese Ausführungsformen sind zur Entfernung von Belag von Oberflächen durch eine Bewegung der Öse 1 etwa parallel über die Zahnoberfläche geeignet, während das Risiko einer Verletzung durch eine nach außen gerichtete Schneidkante oder Spitze reduziert ist.

Die Figuren 11 bis 15 zeigen eine Fortbildung der Ausführungsformen von Figuren 6 bis 10 und weisen zumindest anteilig zwei beabstandete äußere Schneidkanten 14 auf, die entlang der Außenfläche 13 der Öse 1 ausgebildet sind. Die beabstandeten äußeren Schneidkanten 14 werden beispielsweise durch eine kegelstumpfförmigen Querschnitt der Öse 1 ausgebildet, dessen der Öffnung der Öse 1 gegenüberliegende umlaufende Schmalseite 15 mit den angrenzenden ebenen oder bogenförmigen Teilflächen 16 die äußeren Schneidkanten 14 bilden.

In Ausführungsformen, in denen die Öse 1 zusätzlich zu Schneidkanten 11, die die Öffnung der Öse 1 begrenzen, äußere Schneidkanten 14 aufweist, die die Schmalseite 15 beidseitig begrenzen, aufweist, sind auch zur Entfernung von Belägen von Zahnoberflächen durch Schieben der Öse 1 in Richtung der Öse 1 etwa parallel zu einer Zahnoberfläche geeignet. Diese äußeren Schneidkanten 14 sind bei Verwendung der Kürette als Stoßinstrument bevorzugt. Auch diese Ausführungsform reduziert das Verletzungsrisiko, da die Schneidkanten keine Spitze aufweisen.

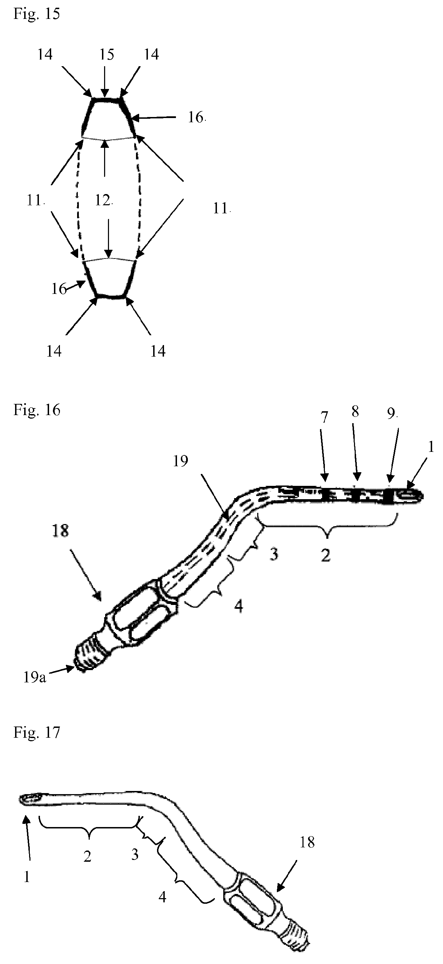

Figur 16 zeigt das Arbeitsende einer Dentalkürette in einer Ausführung als Ultraschallspitze bzw. Schallspitze (mit einer Neigung nach rechts) mit einer distal am Träger angeordneten Öse 1, die mit einem Schaftabschnitt 2 verbunden ist. Gegenüber der Öse 1 ist der Schaftabschnitt 2 in einem stumpfen Winkel von ca. 120° mit einem Winkelabschnitt 3 verbunden, der in einen Distanzabschnitt 4 übergeht, wobei vorliegend der Winkelabschnitt 3 im Wesentlichen linear oder in einem leichten Bogen in den Distanzabschnitt 4 übergeht. Der Distanzabschnitt 4 ist gegenüber der Öse 1 mit einem Verbindungsstück 18 verbunden, das ein Gewinde aufweist, mittels dessen der Träger mit dem Schallhandstück bzw. Ultraschallhandstück, insbesondere mit dem Vibrationserzeuger 17, schwingungsfest verbunden werden kann. Ein Wasserführungskanal 19 verläuft längs des Trägers, der in der gezeigten Ausführungsform aus Distanzabschnitt 4, Winkelabschnitt 3, Schaftabschnitt 2 und Verbindungssstück 18 besteht. Der Wasserführungskanal 19 mündet innerhalb der Öse 1. An seinem der Mündung gegenüberliegenden Ende weist der Wasserführungskanal 19 einen Anschluss 19a für den Zulauf von Wasser auf.

Figur 16 zeigt die bevorzugten Markierungen des Schaftabschnitts 2 anhand einer ersten Markierung 7 sowie einer zweiten Markierung 8 und einer dritten Markierung 9, die insbesondere Eindringtiefen für Zahnfleischtaschen verschiedener Zähne gemäß der WHO-Vorschrift zur Sondeneinteilung anzeigen.

Figur 17 zeigt ein weiteres Arbeitsende einer Dentalkürette in einer Ausführung als Ultraschallspitze bzw. Schallspitze (mit einer Neigung nach links), bei der der Träger anschließend an eine am distalen Ende angeordnete Öse 1 einen Schaftabschnitt 2 aufweist, der in einem stumpfen Winkel mit einem Winkelabschnitt 3 verbunden ist. Der Winkelabschnitt 3 geht in einem im Wesentlichen gestreckten Winkel in den Distanzabschnitt 4 über, an dem wiederum gegenüber dem Winkelabschnitt 3 ein Verbindungsstück 18 angeordnet ist, wobei das Verbindungsstück 18 dazu eingerichtet ist, eine kraftschlüssige und/oder formschlüssige Verbindung mit einem Vibrationserzeuger 17 einzugehen. In der gezeigten Ausführungsform, ist der Träger massiv und weist keinen Wasserführungskanal 19 auf.

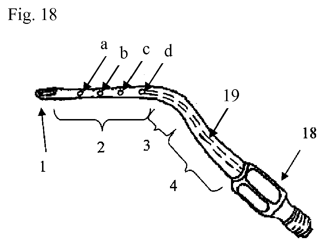

Figur 18 zeigt ein weiteres Arbeitsende einer Dentalkürette in einer Ausführung als Schallspitze bzw. Ultraschallspitze mit einer distal am Träger angeordneten Öse 1, die mit einem Schaftabschnitt 2 verbunden ist. Angrenzend an den Schaftabschnitt 2 weist die Dentalkürette einen Winkelabschnitt 3 auf, der an seinem dem Schaftabschnitt 2 gegenüberliegenden Ende mit einem Distanzabschnitt 4 verbunden ist. Der Schaftabschnitt 2 und der Winkelabschnitt 3 sind in einem stumpfen Winkel miteinander verbunden, der Winkelabschnitt 3 und der Distanzabschnitt 4 sind in einem im Wesentlichen gestreckten Winkel miteinander verbunden. Gegenüber dem Winkelabschnitt 3 ist an dem Distanzabschnitt 4 ein Verbindungsstück 18 mit einem Gewinde angeordnet, über das der Träger mit dem Schallhandstück, insbesondere mit dem Vibrationserzeuger 17, schwingungsfest verbunden werden kann. Ein Wasserführungskanal 19 verläuft längs des Trägers, der in der gezeigten Ausführungsform, aus Distanzabschnitt 4, Winkelabschnitt 3, Schaftabschnitt 2 und Verbindungssstück 18 besteht, und mündet innerhalb des Schaftabschnitts 2. Die Positionen a, b, c und d markieren beispielhaft mögliche Positionen für Mündungsöffnungen des Wasserkanals 19 innerhalb des Schaftabschnitts 2. Die Mündungsöffnungen innerhalb des Schaftabschnitts 2 können dabei vollumfänglich im Bereich des Schaftabschnitts 2 angeordnet sein, z.B. auf der Seite des Schaftabschnitts 2, an der der Distanzabschnitt und der Winkelabschnitt in einem stumpfen Winkel mit dem Schaftabschnitt verbunden sind (gezeigt), oder auf der Seite des Schaftabschnitts 2, an der der Distanzabschnitt und der Winkelabschnitt in einem überstumpfen Winkel mit dem

Schaftabschnitt verbunden sind (nicht gezeigt) und/oder auf einer oder beiden der dazwischenliegenden Seiten angeordnet sein.

An seinem der Mündung gegenüberliegenden Ende weist der Wasserführungskanal 19 einen Anschluss 19a für den Zulauf von Wasser auf.

## Patentansprüche

1. Zahnmedizinische Kürette mit einem stabförmigen Griff (5) und zumindest einem endständig angeordneten Träger, der distal gegenüber dem Griff (5) eine Schneidkante (11) aufweist, wobei der Träger einen Distanzabschnitt (4), einen gegenüber dem Griff (5) am Distanzabschnitt (4) angeordneten Winkelabschnitt (3) und einen am Winkelabschnitt (3) in einem stumpfen Winkel angeordneten Schaftabschnitt (2) aufweist, der gegenüber dem Winkelabschnitt (3) mit einer Öse (1) verbunden ist, die zumindest an einer Kante, die ihre Öffnung begrenzt, die Schneidkante (11) aufweist, wobei der Winkelabschnitt (3) und der Schaftabschnitt (2) in einer Längsmittelebene entlang der Längsachse des Griffs (5) angeordnet sind, **dadurch gekennzeichnet, dass** die Öse (1) in einer Ebene aufgespannt ist, die in einem Winkel von 30 bis 60° zur Längsmittelebene geneigt ist.

2. Zahnmedizinische Kürette nach Anspruch 1, **dadurch gekennzeichnet, dass** der Griff (5) einen Vibrationserzeuger (17) aufweist, der schwingungsfest mit dem Träger verbunden ist.

3. Zahnmedizinische Kürette nach Anspruch 2, **dadurch gekennzeichnet, dass** der Träger (5) ein Befestigungsstück (18) aufweist, das mit dem Vibrationserzeuger (17) schwingungsfest verbindbar ist.

4. Zahnmedizinische Kürette nach Anspruch 2 oder 3, **dadurch gekennzeichnet, dass** entlang des Griffs (5), des Distanzabschnitts (4), des Winkelabschnitts (3) und des Schaftabschnitts (2) ein Wasserführungskanal (19) angeordnet ist, dessen Mündung innerhalb der Öse (1) oder im Schaftabschnitt (2) angeordnet ist und der gegenüber seiner Mündung einen Anschluß (19a) für Wasser aufweist.

5. Zahnmedizinische Kürette nach einem der voranstehenden Ansprüche, **dadurch gekennzeichnet, dass** die Öse (1) jeweils eine umfänglich geschlossene Schneidkante (11) aufweist, die eine Seite der Öffnung der Öse (1) begrenzt.

6. Zahnmedizinische Kürette nach einem der voranstehenden Ansprüche, **dadurch gekennzeichnet, dass** der Griff (5) an jedem Ende einen Träger mit einer Öse (1) aufweist, die in zueinander parallelen Ebenen angeordnet und zur Längsmittelebene geneigt sind.

7. Zahnmedizinische Kürette nach einem der voranstehenden Ansprüche, **dadurch gekennzeichnet, dass** entlang des Griffs (5), des Distanzabschnitts (4), des Winkelabschnitts (3) und des Schaftabschnitts (2) ein Saugkanal (10) angeordnet ist, dessen Mündung innerhalb der Öse (1) angeordnet ist und mit einer von einem Finger zu überdeckenden Öffnung (6) verbunden ist, die im Griff (5) oder im Distanzabschnitt (4) angeordnet ist, wobei der Saugkanal (10) gegenüber seiner Mündung einen Anschluss (10a) für einen Saugschlauch aufweist.

8. Zahnmedizinische Kürette nach einem der voranstehenden Ansprüche, **dadurch gekennzeichnet, dass** der Distanzabschnitt (4) in einem Winkel zum Winkelabschnitt (3) angeordnet ist und die Öse (1) die Längsmittelachse des Griffs (5) umfasst.

9. Zahnmedizinische Kürette nach einem der voranstehenden Ansprüche, **dadurch gekennzeichnet, dass** die Öse (1) einen Querschnitt aufweist, dessen Außenflächen (13) bogenförmig konvex sind mit einer Innenfläche (12), die in einem Winkel von 70 bis 110° an die Außenfläche (13) angrenzt und dort die Schneidkante (11) bildet.

10. Zahnmedizinische Kürette nach einem der voranstehenden Ansprüche, **dadurch gekennzeichnet, dass** die Öse (1) zwei beabstandete äußere Schneidkanten (14) aufweist, die durch Schmalseiten (15) eines kegelstumpfförmigen oder tropfenförmigen Querschnitts der Öse (1) und daran anstoßende Teilflächen (16) gebildet werden.

11. Zahnmedizinische Kürette nach einem der voranstehenden Ansprüche, **dadurch gekennzeichnet, dass** die Innenfläche (12) der Öse (1) konkav ist.

12. Zahnmedizinische Kürette nach einem der voranstehenden Ansprüche, **dadurch gekennzeichnet, dass** der Träger und die Öse (1) einstückig ausgebildet sind.

13. Zahnmedizinische Kürette nach einem der voranstehenden Ansprüche, **dadurch gekennzeichnet, dass** die Öse (1) auf der Längsachse des Griffs (5) angeordnet ist.

14. Zahnmedizinische Kürette nach einem der voranstehenden Ansprüche, **dadurch gekennzeichnet, dass** die Öse (1) einen Außendurchmesser von 2 bis 5 mm aufweist und einen Querschnitt, der in Richtung der Öffnungsebene der Öse einen Abstand der Außenfläche (13) zur Schneidkante (11) von 0,3 bis 1 mm, bevorzugt 0,5 mm aufweist.

## Claims

1. Dental curette having a rod-shaped handle (5) and at least one terminally arranged carrier which distally opposite of the handle (5) has a cutting edge (11), wherein the carrier has a spacer section (4), an angular segment (3) arranged on the spacer section (4) opposite the handle (5) and a shaft segment (2) arranged in an obtuse angle on the angular segment (3), which opposite to the angular segment (3) is connected with an eyelet (1), which at least on an edge confining its opening has the cutting edge (11), wherein the angular segment (3) and the shaft segment (2) are arranged in a longitudinal central plane along the longitudinal axis of the handle (5), **characterized in that** the eyelet (1) is opened up in a plane which is inclined in an angle of 30 to 60° to the longitudinal central plane.

2. Dental curette according to claim 1, **characterized in that** the handle (5) has a vibration generator (17) which is coupled to the carrier in a vibration proof manner.

3. Dental curette according to claim 2, **characterized in that** the carrier (5) has a fixing piece (18), which can be coupled to the vibration generator (17) in a vibration proof manner.

4. Dental curette according to claim 2 or 3, **characterized in that** along the handle (5), the spacer segment (4), the angular segment (3) and the shaft segment (2) there is arranged a water conducting channel (19), the mouth of which is arranged within the eyelet (1) or within the shaft segment (2), and which opposite its mouth has a connection (19)a for water.

5. Dental curette according to one of the preceding claims, **characterized in that** the eyelet (1) has a circularly closed cutting edge (11) each, which confines one side of the opening of the eyelet (1).

6. Dental curette according to one of the preceding claims, **characterized in that** the handle (5) on each end has a carrier with an eyelet (1) which are arranged in planes parallel to one another and which are inclined to the longitudinal central plane.

7. Dental curette according to one of the preceding claims, **characterized in that** along the handle (5), the distance segment (4), the angular segment (3) and the shaft segment (2) there is arranged a suction channel (10), the mouth of which is arranged within the eyelet (1) and is connected to an opening (6) that can be covered by a finger, the opening (6) being arranged within the handle (5) or within the distance segment (4), wherein the suction channel (10) opposite of its mouth has a connection (10a) for a suction tubing.

8. Dental curette according to one of the preceding claims, **characterized in that** the distance segment (4) is arranged in an angle to the angular segment (4) and the eyelet (1) surrounds the longitudinal central axis of the handle (5).

9. Dental curette according to one of the preceding claims, **characterized in that** the eyelet (1) has a cross-section, the outer surfaces (13) of which are arcuately convex having an inner surface (12) which borders on the outer surface (13) in an angle of 70 to 110°, there forming the cutting edge (11).

10. Dental curette according to one of the preceding claims, **characterized in that** the eyelet (1) has two spaced-apart outer cutting edges (14), which are formed by the narrow sides (15) of a frustrum-shaped or drop-shaped cross-section of the eyelet (1) and partial surfaces bordering thereat.

11. Dental curette according to one of the preceding claims, **characterized in that** the inner surface (12) of the eyelet (1) is concave.

12. Dental curette according to one of the preceding claims, **characterized in that** the carrier and the eyelet (1) are formed as one piece.

13. Dental curette according to one of the preceding claims, **characterized in that** the eyelet (1) is arranged on the longitudinal axis of the handle (5).

14. Dental curette according to one of the preceding claims, **characterized in that** the eyelet (1) has an outer diameter of 2 to 5 mm and a cross-section which in the direction of the opening plane of the eyelet has a distance of the outer surface (13) to the cutting edge (11) of 0.3 to 1 mm, preferably of 0.5 mm.

## Revendications

1. Curette dentaire ayant une poignée en forme de tige (5) et au moins un support disposé en une extrémité, présentant un bord tranchant (11) distalement par rapport à la poignée (5), où le support présente une section d'entretoise (4), une section angulaire (3) disposée en face de la poignée (5) au niveau de la section d'entretoise (4) et une section de tige (2) disposée au niveau de la section angulaire (3) dans un angle tronqué, section de tige reliée par un oeillet (1) par rapport à la section angulaire (3), oeillet présentant au moins en une tranche limitant son ouverture le bord tranchant (11), où la section angulaire (3) et la section de tige (2) sont agencées dans un plan médian longitudinal le long de l'axe longitudinal de la poignée (5), **caractérisé en ce que** l'oeillet (1) est tendu dans un plan qui est incliné dans un angle de 30 à 60° par rapport au plan médian longitudinal.

2. Curette dentaire selon la revendication 1, **caractérisée en ce que** la poignée (5) présente un générateur de vibrations (17), qui est relié au support de façon à résister aux oscillations.

3. Curette dentaire selon la revendication 2, **caractérisée en ce que** le support (5) présente une pièce de fixation (18) qui peut être reliée de façon à résister aux oscillations au générateur de vibrations (17).

4. Curette dentaire selon les revendications 2 ou 3, **caractérisée en ce qu'**un canal d'arrivée d'eau (19) est aménagé le long de la poignée (5), de la section d'entretoise (4), de la section angulaire (3) et de la section de tige (2), dont l'embouchure est disposé à l'intérieur de l'oeillet (1) ou bien dans la section de tige (2) et présente un raccord (19a) pour l'eau en face de son embouchure.

5. Curette dentaire selon l'une des revendications précédentes, **caractérisée en ce que** l'oeillet (1) présente respectivement un bord tranchant (11) fermé sur son pourtour, bord limitant un côté de l'ouverture de l'oeillet (1).

6. Curette dentaire selon l'une des revendications précédentes, **caractérisée en ce que** la poignée (5) présente à chaque extrémité un support avec un oeillet (1), supports disposés sur des plans parallèles l'un à l'autre et inclinés par rapport au plan médian longitudinal.

7. Curette dentaire selon l'une des revendications précédentes, **caractérisée en ce qu'**un canal d'aspiration (10) est aménagé le long de la poignée (5), de la section d'entretoise (4), de la section angulaire (3) et de la section de tige (2), dont l'embouchure est disposée à l'intérieur de l'oeillet (1) et qui est reliée à une ouverture (6) à recouvrir d'un seul doigt, ouverture disposée dans la poignée (5) ou bien dans la section d'entretoise (4), où le canal d'aspiration (10) présente un raccord (10a) pour tuyau d'aspiration en face de son embouchure.

8. Curette dentaire selon l'une des revendications précédentes, **caractérisée en ce que** la section d'entretoise (4) est disposée à un angle par rapport à la section angulaire (3) et l'oeillet (1) comporte l'axe médian longitudinal de la poignée (5).

9. Curette dentaire selon l'une des revendications précédentes, **caractérisée en ce que** l'oeillet (1) présente une section transversale dont les surfaces externes (13) sont convexes en arc-de-cercle avec une surface interne (12), touchant la surface externe (13= à un angle de 70° à 110° et constitue à cet endroit le bord tranchant (11).

10. Curette dentaire selon l'une des revendications précédentes, **caractérisée en ce que** l'oeillet (1) présente deux bords tranchants externes espacés (14) qui sont constituées par des petits côtés (15) d'une section transversale de l'oeillet (1) de forme tronconique ou en forme de goutte, et de faces partielles (16) butant contre ces derniers.

11. Curette dentaire selon l'une des revendications précédentes, **caractérisée en ce que** la surface interne (12) de l'oeillet (1) est concave.

12. Curette dentaire selon l'une des revendications précédentes, **caractérisée en ce que** le support et l'oeillet (1) forment un seul bloc.

13. Curette dentaire selon l'une des revendications précédentes, **caractérisée en ce que** l'oeillet (1) est disposé sur l'axe longitudinal de la poignée (5).

14. Curette dentaire selon l'une des revendications précédentes, **caractérisée en ce que** l'oeillet (1) présente un diamètre extérieur de 2 à 5 mm et un diamètre qui présente un intervalle, entre la surface externe (13) et le bord tranchant (11), de 0,3 à 1 mm, de préférence 0,5 mm dans la direction du plan d'ouverture.
